# EUROPÄISCHE PATENTSCHRIFT

(11) **EP 0 568 987 B1**
(45) Veröffentlichungstag und Bekanntmachung des Hinweises auf die Patenterteilung: **09.07.1997**
(21) Anmeldenummer: 93107252.4
(22) Anmeldetag: 05.05.1993
(51) Int. Cl.: B65D 5/42, B65D 77/04, A61M 5/14

(54) **Faltschachtel zum Beispiel für Infusionsflaschen**
Collapsable box, e.g. for infusion bottles
Boîte pliable, par exemple pour des flacons de perfusion

(30) Priorität: 06.05.1992 DE 4214416
(43) Veröffentlichungstag der Anmeldung: 10.11.1993
(73) Patentinhaber: BYK GULDEN LOMBERG CHEMISCHE FABRIK GMBH, D-78403 Konstanz (DE)
(72) Erfinder: Gräber, Iris, W-7760 Radolfzell (DE); Dybowski, Ulrich, Dr., W-7750 Konstanz (DE); Lippert, Rita, W-7753 Allensbach (DE)

(56) Entgegenhaltungen:
- DE-U- 8 604 054
- US-A- 2 033 401
- US-A- 3 819 036
- US-A- 4 166 533

## Beschreibung

### Technisches Gebiet

Die Erfindung betrifft eine Faltschachtel für einen über Kopf aufzuhängenden flaschenartigen Behälter.

### Stand der Technik

Die Notwendigkeit, flaschenartige Behälter über Kopf aufzuhängen, besteht in vielen Bereichen des täglichen Lebens. Insbesondere finden über Kopf aufgehängte flaschenartige Behälter im medizinischen Bereich Verwendung, beispielsweise bei der Infusion von Medikamenten, Diagnostika, Nährlösungen und dergleichen. Dabei ist es wesentlich, daß die flaschenartigen Behälter einfach und sicher aufgehängt und in aufgehängtem Zustand leicht und einwandfrei gehandhabt werden können.

Es sind zahlreiche Vorrichtungen zum Überkopfaufhängen von flaschenartigen Behältern bekannt.

Die US-A-2 033 401 beschreibt eine gattungsgemäße Faltschachtel gemäß Oberbegriff des Anspruchs.

So beschreibt das DE-GM 7917315 eine spiralförmige Aufhängevorrichtung. Diese Vorrichtung umfaßt einen Haltering für den Flaschenhals und einen Haltering für den Flaschenbauch, die im Lagerzustand in einer Ebene angeordnet sind, wobei der Haltering für den Flaschenhals von dem Haltering für den Flaschenbauch umgeben ist, die beiden Ringe über zwei Stege verbunden sind und der Haltering für den Flaschenbauch eine ebenfalls im Lagerzustand in der Ebene liegende Aufhängeeinrichtung aufweist, die im Lagerzustand ihrerseits den Haltering für den Flaschenbauch umgibt und mit diesem mittels Stegen verbunden ist. Im Gebrauchszustand wird die Flasche mit dem Hals durch den Haltering für den Flaschenhals gesteckt und die Vorrichtung so über die Flasche gezogen, daß der Haltering für den Flaschenbauch den Flaschenbauch umgibt und die Aufhängeeinrichtung zum Aufhängen frei bleibt. Die Vorrichtung umgibt im Gebrauchszustand die Flasche spiralförmig.

Eine weitere Vorrichtung ist in dem DE-GM 7442831 beschrieben und weist jeweils einen Haltering für den Hals einer Flasche und einen Haltering für den Bauch einer Flasche auf, wobei zusätzlich noch zwei Aufhängeösen vorgesehen sind, die am Ring für den Flaschenbauch eingreifen.

Ähnliche Flaschenaufhängevorrichtungen sind in den DE-OS 2128993, DE-OS 2548726 und DE-OS 3310841 beschrieben, die als einstückige Kunststofformteile mit einem dem Flaschenhals und einem dem Flaschenumfang angepaßten Halteteil ausgebildet sind, wobei sich an dem dem Flaschenumfang angepaßten Halteteil ein dieses umgebendes Aufhängeteil anschließt.

Allen diesen Lösungen sind die Nachteile gemeinsam, daß sie material- und fertigungstechnisch aufwendig herzustellen sind und zur Aufhängung selbst eine Aufhängevorrichtung als separates Teil benötigen, die in der Regel schlecht handhabbar ist. Außerdem ist ein zusätzliches Teil, meist ein Kunststoffteil, separat zu entsorgen.

### Beschreibung der Erfindung

Aufgabe der vorliegenden Erfindung ist die Zurverfügungstellung einer Aufhängevorrichtung für über Kopf aufzuhängende flaschenartige Behälter, die einfach zu handhaben ist, leicht zu entsorgen ist und fertigungstechnisch ohne besonderen Aufwand herzustellen ist, ohne daß zusätzliches Material eingesetzt werden muß.

Gelöst wird diese Aufgabe durch eine Faltschachtel für über Kopf aufzuhängende flaschenartige Behälter dadurch, daß zwei sich gegenüber liegende Seitenteile der Faltschachtel jeweils eine um die anliegende Kante des Deckels der Faltschachtel herausklappbare Laschen mit endständigen Aufhängeösen beinhalten.

Die Faltschachtel wird auf übliche Weise aus den für solche Faltschachteln üblichen Materialien hergestellt. Die aus den Seitenteilen der Faltschachtel herausklappbaren Laschen werden vorgeformt, indem deren Umrisse in die Seitenteile der Faltschachtel unter Stehenlassen schmaler Stege ausgestanzt oder durch entsprechende Perforationen festgelegt werden. Die nach dem Herausklappen als Aufhängeösen dienenden Öffnungen werden ausgestanzt. Um das Herauslösen der Laschen zu erleichtern, können die Laschen kopfseitig leicht eingekürzt sein, so daß eine Öffnung entsteht, die es dem Anwender erlaubt eine obere Kante der Lasche zwischen Fingerspitze und Fingernagel zu schieben. Der flaschenartige Behälter wird mit der Kopfseite voraus in die Faltschachtel eingeführt. Nach Verschließen des Deckels der Faltschachtel ist diese einsatzbereit.

Vor der Anwendung werden die beiden Laschen aus den Seitenteilen der Faltschachtel gelöst und nach unten um die anliegende Kante des Deckels der Faltschachtel geklappt. An den beiden Aufhängeösen der Laschen wird die Faltschachtel über Kopf an einem entsprechenden Haken aufgehängt. Durch die nach dem Herausklappen der Laschen entstehenden länglichen Fenster in den gegenüberliegenden Seitenteilen der Faltschachtel läßt sich der Füllstand des flaschenartigen Behälters beobachten. Nach Entnahme des Inhalts des flaschenartigen Behälters wird dieser aus der Faltschachtel entnommen. Die Entsorgung der Faltschachtel und des flaschenartigen Behälters erfolgen auf dem üblichen Weg. Das gesonderte Entsorgen von zusätzlichen Aufhängevorrichtungen entfällt.

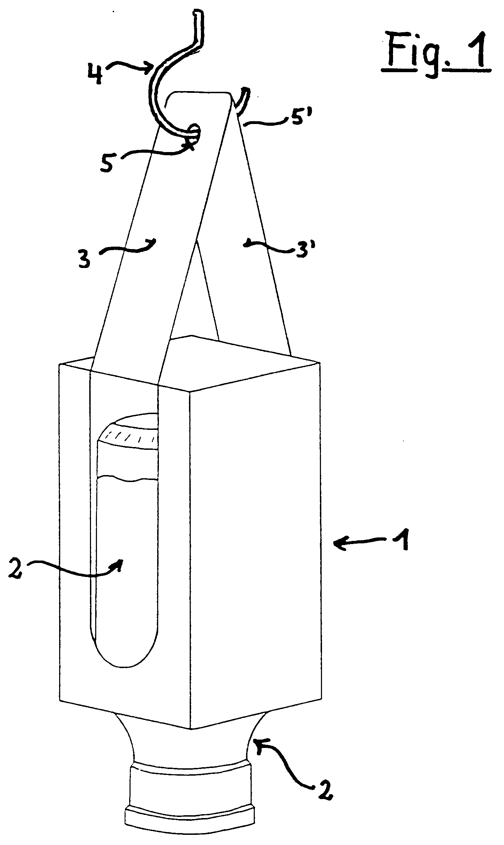

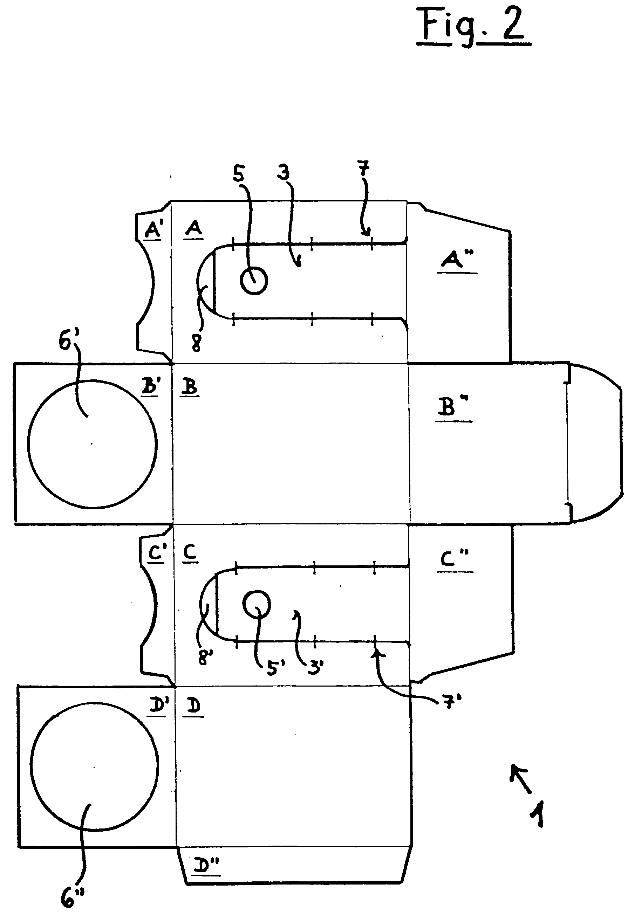

Nachstehend wird die Erfindung anhand der Fig. 1 und 2 näher erläutert.
- Fig. 1: zeigt schematisch eine perspektivische Ansicht einer aufgehängten Faltschachtel mit flaschenartigem Behälter.
- Fig. 2: zeigt den Plan der ungefalteten Faltschachtel.

Bei der in Fig. 1 dargestellten Faltschachtel 1, die einen flaschenartigen Behälter 2 enthält, sind die beiden Laschen 3, 3' aus den Seitenteilen der Faltschachtel herausgeklappt und durch die Aufhängeösen 5, 5' an einem Haken 4 aufgehängt. Die Entnahmeöffnung des flaschenartigen Behälters ragt nach unten durch den kreisrunden Durchbruch 6.

Der in Fig. 2 dargestellte Plan der Faltschachtel 1 zeigt die Seitenteile A, B, C, D mit den anhängenden Klappdeckelteilen A'', B'', C'', den Bodenteilen A', B', C', D' und der mit dem Seitenteil A unlösbar zu verbindenden Lasche D''. Im zusammengeklappten Zustand kommen die beiden Bodenteile B', D', die zentrische Öffnungen 6', 6'' aufweisen, so aufeinander zu liegen, daß die zentrischen Öffnungen 6', 6'' zur Deckung kommen und so den kreisrunden Durchbruch 6 bilden. Die beiden Bodenteile B', D' werden unlösbar miteinander verbunden. Die Laschen 3, 3' sind in den Seitenteilen A, C vorgestanzt. Nach Aufbrechen der mit 7, 7' bezeichneten, beim Stanzen stehengelassenen Stege können die Laschen 3, 3' um die gefalzten Verbindungslinien zwischen den Seitenteilen A bzw. C und den Klappdeckelteilen A'' bzw. C'' geklappt werden. Die Eingriffsöffnungen 8, 8' erleichtern das Loslösen der Laschen 3, 3' aus den Seitenteilen A, C.

## Patentansprüche

1. Faltschachtel für über Kopf aufzuhängende flaschenartige Behälter mit einem Bodenteil, der einen zentrischen kreisrunden Durchbruch (6) aufweist, dessen Durchmesser geringer ist, als der größte Durchmesser des flaschenartigen Behälters (2), dadurch gekennzeichnet, daß zwei sich gegenüber liegende Seitenteile (A, C) der Faltschachtel (1) jeweils eine um die anliegende Kante des Deckels der Faltschachtel (1) herausklappbare Laschen (3, 3') mit endständigen Aufhängeösen (5, 5') beinhalten.

## Claims

1. Folding box for bottle-like containers which are to be suspended overhead and have a base part which has a central, circular through-passage (6) whose diameter is smaller than the largest diameter of the bottle-like container (2), characterized in that two mutually opposite side parts (A, C) of the folding box (1) each contain a tab (3, 3') which can be folded out around the adjoining edge of the lid of the folding box (1) and has a suspension eyelet (5, 5') at its end.

## Revendications

1. Boîte pliante pour des récipients en forme de bouteilles devant être suspendus tête en bas, comportant un élément de fond qui est pourvu d'un passage (6) central circulaire dont le diamètre maximal est inférieur à celui du récipient en forme de bouteille, caractérisé par le fait que deux éléments de côté (A,C) en vis-à-vis comprennent chacun une patte (3,3') pourvue d'un trou d'accrochage (5,5') à son extrémité, qui peut être rabattue autour du bord contigu du couvercle de la boîte pliante (1).
